# EUROPEAN PATENT APPLICATION

(11) **EP 0 630 631 A2**
(43) Date of publication of application: **28.12.1994**
(21) Application number: 94304543.5
(22) Date of filing: 22.06.1994
(51) Int. Cl.: A61F 13/15

(54) **Disposable undergarment**

(30) Priority: 22.06.1993 JP 33611/93
(71) Applicant: UNI-CHARM CORPORATION, Kawanoe-shi Ehime-ken (JP)
(72) Inventor: Yamamoto, Masamitsu, Kawanoe-shi, Ehime-ken (JP); Hisada, Kenichi, Kawanoe-shi, Ehime-ken (JP)
(74) Representative: Warren, Anthony Robert

(57) **Abstract**

A disposable undergarment comprises a fibrous nonwoven fabric stretchable in length and width, having a stretching stress higher than 30 g in width, is used as a basic cloth (1). An absorbent pad (10), smaller than the basic cloth, is bonded to the inner surface thereof. The absorbent pad (10) has a middle zone (S₁) with a higher rigidity than opposite side zones (S₂).

## Description

The present invention relates to a disposable undergarment of the pants type formed by bonding front and rear bodies along respective opposite side edges at waist level, such as incontinence diapers, baby diapers, baby training pants and sanitary pants.

Such an undergarment of the prior art usually employs nonelastic nonwoven fabric as a base cloth, and elastic members each comprising a plurality of thread-like elastic elements or a single tape-like elastic member bonded, after being stretched by a predetermined elongation percentage, to respective leg-openings along peripheral edges thereof by means of hot melt type adhesive in order to keep the leg openings fitting around the wearer's legs and thereby to avoid excretion leakage possibly occurring around the legs.

However, use of said elastic members sometimes causes pressure marks around the wearer's legs or even causes pain to the wearer.

While it may be contemplated, as one measure to produce the undergarment at a low cost, to arrange the undergarment using none of said elastic members so that excretion leakage occurring around the wearer's legs may be avoided, such an arrangement has not been realized.

It is an object of the invention to eliminate the abovementioned problems by a novel arrangement of a basic cloth and an absorbent element as will be described in detail.

The object set forth above is achieved, according to the invention, by a disposable undergarment comprising a basic cloth made of fibrous nonwoven fabric elastically stretchable at least in length and width and provided with a waist-opening and a pair of leg-openings, wherein fibrous nonwoven fabric having a stretching stress of at least 30 g, at least in width, is used as said basic cloth and an absorbent pad has a smaller size than that of said basic cloth so that outer peripheral edges of said basic cloth may extend outward from those of said absorbent pad and is bonded, preferably intermittently, to the inner surface of said basic cloth, and wherein said absorbent pad has a rigidity lower than 7 g/cm and this rigidity gradually decreases from a longitudinally middle zone to longitudinally opposite end zones of said absorbent pad.

Inspite of the fact that the leg-openings are not provided with elastic members, peripheral edges of the respective leg-openings fit well around the wearer's legs.

The invention will be described by way of example with reference to the accompanying drawings, in which:
Fig. 1 is a front view showing a first embodiment of an undergarment constructed according to the invention;
Fig. 2 is a developed plan view showing, partially broken away, the inside of said undergarment;
Fig. 3 is an enlarged scale sectional view showing an absorbent pad; and
Fig. 4 is an enlarged scale sectional view taken along a
   line X-X in Fig. 2.

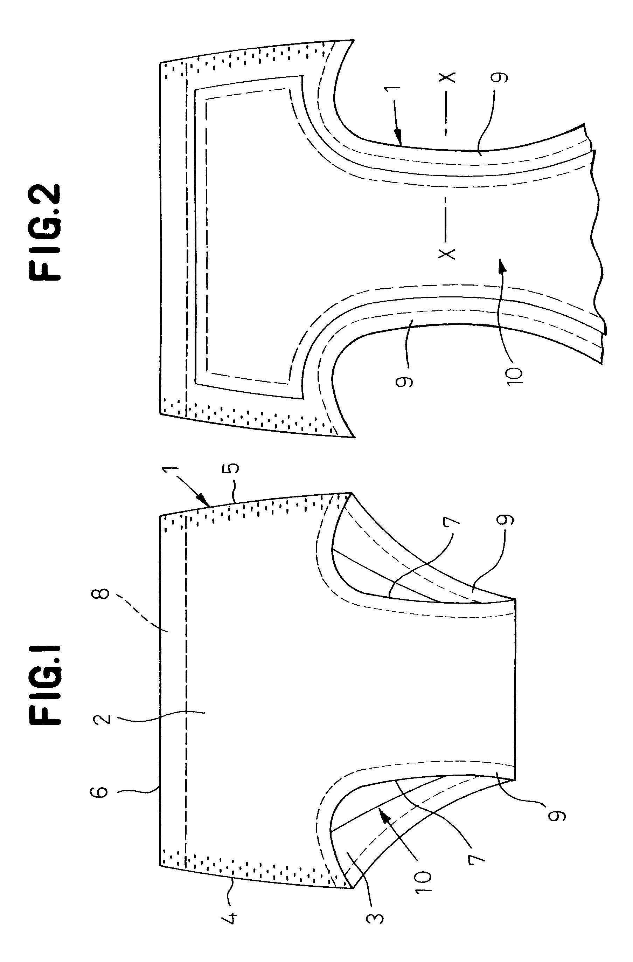

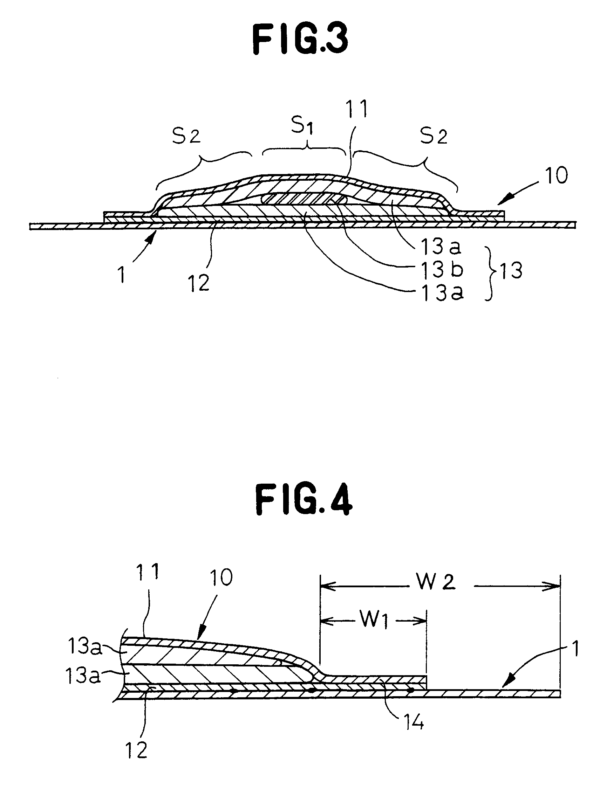

Referring to Figs. 1 through 4, there is shown an embodiment of the invention. An undergarment is cut out from fibrous nonwoven fabric used as a basic cloth 1 having an elastic stretchability both in length and width (preferably, an elastic stretchability higher in width than in length) and has a waist-opening 6 and a pair of leg-openings 7 formed by bonding opposite side edges 4, 5 of a front body 2 to corresponding opposite side edges 4, 5 of a rear body 3 in dotted pattern.

The basic cloth 1 of the front and rear bodies 2, 3 may be formed, for example, from a web of heat crimped synthetic fibre having its component fibres entangled or intertwined by subjecting them to fluid jet or intermittently bonding together under pressure and heat. The basic cloth 1 used herein has a stretching stress in length and width of 30 g or higher, more preferably, of 50 to 130 g.

The waist-opening 6 is provided on its inner peripheral surface with an elastic member 8 in the form of tape so as to be elastically stretchable in the circumferential direction, but the leg-openings 7 have none of the elastic members as have conventionally been attached to the leg-openings. It should be understood that, if desired, suitable synthetic resin of hot melt type having a rubber-like elasticity in its cured state, which has been conventionally employed for bonding members of a disposable diaper, may be applied to inner or outer peripheral surfaces 9 of each leg-opening 7, so as to define a ribbon-like zone, so that the region of each leg-opening may have a stretching stress higher than that in the remaining region. It is also possible to use, instead of said resin, so-called rubber elastomer which is an elastic material in the form of a liquid or gel presenting a rubber-like elasticity in its cured state or after heat treatment.

An absorbent pad 10 is laid upon the inner surface of the basic cloth 1. The absorbent pad 10 has a contour substantially similar to that of the basic cloth 1 and a size smaller than that of the basic cloth 1. The absorbent pad 10 comprises liquid-permeable top- and backsheets 11, 12 both made of fibrous nonwoven fabric,and a liquid-absorbent core 13 sandwiched therebetween,wherein regions 14 each having a width W₁ of the top- and backsheets extending outward from the outer peripheral edge of the core 13 are bonded together by means of adhesive or suitable welding means. The backsheet 12 may be also formed, if desired, by a liquid-impermeable sheet such as water-proofed fibrous nonwoven fabric or plastic film.

Sandwiched between the top- and backsheets 11, 12, the core 13 may be divided into a longitudinally middle zone S₁ and longitudinally opposite side zones S₂, for better understanding the specific structure thereof. The middle zone S₁ has a rigidity higher than that in the opposite side zones S₂. Each side zone S₂ has a rigidity (Taber stiffness) lower than 7 g/cm according to the TAPII standard, preferably of 5 to 2 g/cm. While a liquid-absorbent layer 13b is interposed between a pair of liquid-absorbent layers 13a both being thicker than said liquid-absorbent layer 13b in order to achieve the above-mentioned rigidity relationship in the specific embodiment illustrated, it is also possible to increase the material density or compress the material and thereby to increase the rigidity in the middle zone S₁. More preferably, the core 13 is shaped to have a gradually decreasing thickness from a region adjacent its outer peripheral edges to its outer peripheral edges,so that an abrupt difference in level which otherwise would appear between the basic cloth 1 and the outer side edges of the absorbent pad 10 may be avoided and, in consequence, a zone adjacent each leg-opening 7 may fit well around the wearer's leg. Such desired shape of the core 13 can be achieved, for example, by appropriate allotment of core materials.

The absorbent pad 10 is intermittently (preferably in dotted pattern) bonded to the basic cloth 1 by means of adhesive or suitable welding means (not shown). A distance (width) W₂ from the inner side edge of each side flap 14 to the corresponding outer side edge of the basic cloth 1 depends on the rigidity of the absorbent pad 10 and is preferably 10 mm or larger to maintain a desired contractile force of the ribbon-like elastic zone 9. Each of the outwardly extending regions 14 preferably has a rigidity lower than 2 g/cm according to said TAPII standard.

With the undergarment constructed as described hereinabove according to the invention, the outer peripheral edges of the respective leg-openings are almost not affected by the rigidity of the absorbent pad. Namely, the outer peripheral edges of the leg-openings fit well around the wearer's legs under a stretching stress of the basic cloth itself,and neither a backward bending nor a disengagement of the outer peripheral edges occurs during use of the undergarment inspite of the fact that said outer peripheral edges have no elastic members. In this manner, the undergarment of the invention is comparable with the conventional undergarment provided with the elastic members for preventive effect against excretion leakage,and superior to the undergarment of prior art in mass-productivity at a low cost.

## Claims

1. A disposable undergarment comprising a basic cloth made of fibrous nonwoven fabric elastically stretchable at least in length and width and provided with a waist-opening and a pair of leg-openings, wherein fibrous nonwoven fabric having a stretching stress of at least 30 g, at least in width, is used as said basic cloth,and an absorbent pad,having a smaller size than that of said basic cloth so that outer peripheral edges of said basic cloth extend outward from those of said absorbent pad, is intermittently bonded to the inner surface of said basic cloth, and wherein said absorbent pad has a longitudinally middle zone and longitudinally opposite side zones and said middle zone has a rigidity higher than that in said opposite side zones.

2. A disposable undergarment accoring to Claim 1, wherein the rigidity of said opposite side zones is lower than 7 g/cm.

3. A disposable undergarment according to Claim 1 or 2, wherein said absorbent pad is shaped to have a thickness gradually decreasing from a region adjacent its opposite side edges to its opposite side edges.
